# EUROPÄISCHE PATENTANMELDUNG

(11) **EP 1 106 068 A2**
(43) Veröffentlichungstag der Anmeldung: **13.06.2001**
(21) Anmeldenummer: 00126053.8
(22) Anmeldetag: 29.11.2000
(51) Int. Cl.: A21D 2/16, A21D 2/18, A21D 6/00, A23L 1/035, A23P 1/06, A61K 9/14, A61K 9/16, B01F 17/00

(54) **Verfahren zur Herstellung von Emulgiermittel enthaltende Pulverprodukte**

(30) Priorität: 08.12.1999 DE 19959036
(71) Anmelder: Cognis Deutschland GmbH, 40589 Düsseldorf (DE)
(72) Erfinder: Horlacher, Peter, Dr., 89079 Ulm (DE); Gölitz, Hartmut, 87787 Wolfertschwenden (DE); Sander, Andreas, Dr., 89257 Illertissen (DE); Adams, Wolfgang, 88074 Meckenbeuren (DE)

(57) **Zusammenfassung**

Vorgeschlagen wird ein Verfahren zur Herstellung eines frei fließfähigen Pulverproduktes, bei dem man eine oberflächenaktive Substanz mit einem partikelförmigen Träger mischt und anschließend vermahlt.

## Beschreibung

### Gebiet der Erfindung

Die Erfindung liegt auf dem Gebiet der Herstellung von Pulverprodukten und betrifft ein Verfahren zur Herstellung von Pulverprodukten aus oberflächenaktiven Substanzen und Trägern.

### Stand der Technik

Oberflächenaktive Substanzen werden in Lebensmitteln sowie in kosmetischen und pharmazeutischen Mittel in einer Vielzahl von Anwendungen und zu einer Vielzahl von Zwecken eingesetzt. Ziel ist meist die Herstellung von Emulsionen. Stellt man Emulsionen her, bei denen Luft die innere (disperse) Phase darstellt, spricht man von sogenannten Aufschlagmitteln. Solche Aufschlagmittel sind beispielsweise in der Lebensmittelindustrie bei der Herstellung von Brotteigen, Kuchenmassen oder Dessertspeisen von Bedeutung. Der Zusatz der oberflächenaktiven Stoffe zu den aufzuschlagenden Mischungen erfolgt dabei vorteilhafter Weise in Form von Pulvern. Es ist bekannt solche Pulver entweder durch Sprühtrocknung der oberflächenaktiven Substanzen herzustellen (wie beispielsweise in **DE 27 19 580** beschrieben) oder durch Aufbringen der oberflächenaktiven Substanz auf einen Träger. So beschreibt das US-Patent **US 3 973 046** ein Verfahren zur Herstellung einer pulverförmigen Mischung, bei dem ein Fett zunächst partiell kristallisiert wird, danach extrudiert wird und das Extrudat dann direkt mit einen Träger gemischt wird. Die europäische Patentschrift **EP 0 153 870 B1** (Nexus APS) beschreibt ein Verfahren, bei dem die oberflächenaktive Substanz mit ausgewählten Trägern vermischt wird, und diese Mischung anschließend bei Temperaturen von 100 bis 180 °C extrudiert wird. Nachteilig an den genannten Methoden sind zum einen der erforderliche hohe apparative Aufwand, sowie die z.T. erhebliche thermische und mechanische Belastung der oberflächenaktiven Substanzen. Diese können zu einem teilweisen Verlust der oberflächenaktiven Eigenschaften führen. Dies ist sowohl aus technologischen als auch aus Kostengründen nicht erwünscht.

Die Aufgabe der vorliegenden Erfindung hat somit darin bestanden, ein Verfahren zur Verfügung zu stellen, welches es ermöglicht eine oberflächenaktive Substanz in Form eines Pulvers zur Verfügung zu stellen, bei dem die thermische Belastung der oberflächenaktiven Substanz sowie der Kostenaufwand des Verfahrens gegenüber bekannten Verfahren deutlich reduziert sind.

### Beschreibung der Erfindung

Überraschender weise wurde gefunden, daß man ein frei fließfähiges Pulverprodukt erhält, indem man eine oberflächenaktive Substanz mit einem partikelförmigen Träger mischt und anschließend vermahlt.

Durch das erfindungsgemäße Verfahren ist es möglich auch thermisch labile (d.h. nicht über eine Temperatur über 100 °C erwärmbare), oberflächenaktive Substanzen einzusetzen. Die so erhaltenen Produkte sind vielfältig einsetzbar, sie zeigen sich sowohl in der Handhabung (Schütt-und Rieselfähigkeit) als auch in der technologischen Leistung (Aufschlagwirkung) mit Produkten des Standes vergleichbar. Neben der geringen thermischen Belastung ist gegenüber den Verfahren des Standes der Technik besonders der geringe erforderliche apparative Aufwand vorteilhaft.

Unter "frei fließfähig" im Sinne der Erfindung werden Pulver verstanden, die bei Raumtemperatur frei rieselfähig sind. Dies hängt von den eingesetzten Komponenten -partikelförmiger Träger sowie oberflächenaktive Substanzen- ab und kann gegebenenfalls durch Zusatz von Rieselhilfen beeinflusst werden. Wenn nicht zusätzliche Rieselhilfen verwendet werden, wird die Fließfähigkeit durch die Größenverteilung der Partikel im Pulver bestimmt. In der Regel liegt bei den erfindungsgemäßen frei fließfähigen Produkten der Anteil von Partikel mit einem Durchmesser kleiner 250 µm bei über 50 %, insbesondere über 75 %. Der Wassergehalt der Produkte beträgt in der Regel unter 10 Gew.-%, insbesondere unter 5 Gew.-%.

### Oberflächenaktive Substanzen

Als oberflächenaktive Substanzen werden alle Substanzen verstanden, die in der Lage sind die Grenzflächenspannung zu beeinflussen. Insbesondere werden darunter Stoffe verstanden, die zur Herstellung und Stabilisierung von Emulsionen geeignet sind. In einer bevorzugten Ausführungsform der vorliegenden Erfindung werden als oberflächenaktive Substanz Stoffe eingesetzt aus der Gruppe der Nahrungsmittelemulgatoren und Emulgatoren für kosmetische und/oder pharmazeutische Mittel.

Je nach Einsatzgebiet kann man zwischen Emulgatoren für Lebensmittel und Emulgatoren für kosmetische und/oder pharmazeutische Mittel unterscheiden. Eine Vielzahl von Emulgatoren ist jedoch für alle genannten Einsatzgebiete geeignet. Die vorliegende Erfindung umfaßt auch die Erkenntnis, daß die Emulgatoren in beliebigen Mischungen untereinander eingesetzt werden können.

### Nahrungsmittelemulgatoren

Als Nahrungsmittelemulgatoren kommen alle für Lebensmittel geeigneten Emulgatoren in Frage. Für eine umfassende Aufzählung dieser Nahrungsmittelemulgatoren wird auf **G. Schuster, Emulgatoren für Lebensmittel, Springer Verlag 1985, S. 55-205** verwiesen.

Beispiele für geeignete Nahrungsmittelemulgatoren sind :
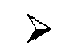 Ester der Diacetylweinsäure mit Fettalkoholen mit 6 bis 22, vorzugsweise 12 bis 18 Kohlenstoff-atomen, wie beispielsweise Diacetylweinsäurecetearylester, aber auch gemischte Ester der Wein- und Essigsäure;
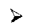 Acyllactate in Form ihrer Alkali- und/oder Erdalkalisalze, wie beispielsweise Natrium- oder Calciumstearoyllactylat,
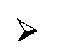 Polysorbate, also Anlagerungsprodukte von 1 bis 20 Mol Ethylenoxid an Sorbitanmono-,-sesqui-, -di-, und/oder -triester auf Basis von Fettsäuren mit 6 bis 22, vorzugsweise 12 bis 18 Kohlenstoff-atomen, wie beispielsweise Addukte von 5 bis 15 Mol Ethylenoxid an Sorbitanmonolaurat, Sorbi-tandipalmitat, Sorbitantristearat, Sorbitanmonooleat und dergleichen;
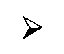 Mono- und Diglyceride sowie deren technische Gemische auf Basis von Fettsäuren mit 6 bis 22, vorzugsweise 12 bis 18 Kohlenstoffatomen, wie beispielsweise Laurinsäuremonoglycerid, Palmi-tinsäuremonoglycerid, Sterarinsäuremonoglycerid oder Ölsäuremonoglycerid sowie deren Anlage-rungsproduke mit 1 bis 20, vorzugsweise 5 bis 10 Mol Ethylenoxid;
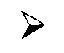 Lecithine
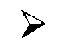 Polyglycerinester (PEG)
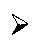 veresterte Monoglyceride
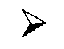 Propylenglykolmonostearat (PGMS)

### Emulgatoren für kosmetische und/oder pharmazeutische Mittel

Als Emulgatoren kommen beispielsweise nichtionogene Tenside aus einer der folgenden Gruppen in Frage:
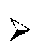 Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
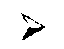 Alkyl- und/oder Alkenyloligoglykoside mit 8 bis 22 Kohlenstoffatomen im Alk(en)ylrest und deren ethoxylierte Analoga;
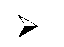 Anlagerungsprodukte von 1 bis 15 Mol Ethylenoxid an Ricinusöl und/oder gehärtetes Ricinusöl;
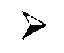 Anlagerungsprodukte von 15 bis 60 Mol Ethylenoxid an Ricinusöl und/oder gehärtetes Ricinusöl;
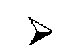 Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
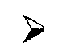 Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
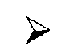 Mischester aus Pentaerythrit, Fettsäuren, Citronensäure und Fettalkohol gemäß **DE 1165574** PS und/oder Mischester von Fettsäuren mit 6 bis 22 Kohlenstoffatomen, Methylglucose und Polyolen, vorzugsweise Glycerin oder Polyglycerin.
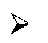 Mono-, Di- und Trialkylphosphate sowie Mono-, Di- und/oder Tri-PEG-alkylphosphate und deren Salze;
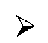 Wollwachsalkohole;
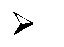 Polysiloxan-Polyalkyl-Polyether-Copolymere bzw. entsprechende Derivate;
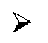 Polyalkylenglycole sowie
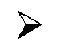 Glycerincarbonat.

Die **Anlagerungsprodukte von Ethylenoxid und/oder von Propylenoxid** an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE 2024051 PS** als Rückfettungsmittel für kosmetische Zubereitungen bekannt.

**Alkyl- und/oder Alkenyloligoglycoside**, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Typische Beispiele für geeignete **Partialglyceride** sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

Als **Sorbitanester** kommen Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

Typische Beispiele für geeignete **Polyglycerinester** sind Polyglyceryl-2 Dipolyhydroxystearate (Dehymuls® PGPH), Polyglycerin-3-Diisostearate (Lameform® TGI), Polyglyceryl-4 Isostearate (Isolan® Gl 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische.

Beispiele für weitere geeignete **Polyolester** sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

Weiterhin können als Emulgatoren **zwitterionische Tenside** verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin.

Schließlich kommen auch **Kationtenside** als Emulgatoren in Betracht, wobei solche vom Typ der Esterquats, vorzugsweise methylquaternierte Difettsäuretriethanolaminester-Salze, besonders bevorzugt sind.

Der Gehalt an oberflächenaktiven Substanzen beträgt in der Regel 10 bis 60 Gew.- % -bezogen auf das Gesamtgewicht der Mischung, insbesondere 20 bis 40 Gew.-%.

Die oberflächenaktive Substanz kann als weitere Bestandteile übliche Verdickern, Gelbildner und/oder Stabilisatoren enthalten. Als Beispiel sei Gelatine genannt.

### Partikelförmiger Träger

Als partikelförmiger Träger kommen alle Substanzen in Betracht, die eine Trägerfunktion für die oberflächenaktive Substanz wahrnehmen und beispielsweise durch Sprühtrocknung oder Mahlen in eine Partikelform überführt wurden. Die Auswahl des partikelförmigen Trägers ist abhängig von der ausgewählten oberflächenaktiven Substanz sowie von dem Gebiet, in dem das Pulverprodukt eingesetzt wird. Üblicherweise weisen die trägerförmigen Partikel folgende Größenverteilung auf: der Hauptteil der Partikel (über 50 %) hat einen Durchmesser im Bereich von 1 bis 250 µm, insbesondere von 1 bis 100 µm. In einer bevorzugten Ausführungsform weisen > 50 % der partikelförmigen Träger eine Größe von 1 bis 75 µm auf.

Die partikelförmigen Träger können sowohl pflanzlichen als auch tierischen bzw. anorganischen Ursprungs sein. Beispiele für Träger tierischen Ursprungs sind Milchprodukte, Beispiele für Träger anorganischen Ursprungs sind Kreiden, Bentonite, Tricaliumphosphat oder Tone. In einer bevorzugten Ausführungsform der vorliegenden Erfindung werden partikelförmige Träger eingesetzt, die pflanzlichen Ursprungs sind. In einer besonders bevorzugten Ausführungsform der vorliegenden Erfindung werden als partikelförmige Träger eingesetzt, die ausgewählt sind aus der Gruppe der pflanzlichen Mehle, pflanzlichen Stärken, Monosaccharide, Disaccharide und Pentosane. Diese sind insbesondere für die Herstellung von pulverförmigen Produkten für die Lebensmittelindustrie von Bedeutung.

Als **pflanzliche Mehle** eignen sich insbesondere Getreidemehle wie Weizen-, Roggen-, Gersten-, Hafer- und Maismehle sowie Leguminosenmehle wie Erbsen-, Bohnen-, Linsen- und Sojamehle.

Als **pflanzliche Stärken** seien genannt Kartoffelstärke, Batatenstärke, Marantastärke, Yamwurzelstärke, Reisstärke, Sagostärke, Maniokstärke, Tapiokastärke (partiell verkleisterte Maniokstärke) sowie Leguminosenstärken wie Erbsen-, Bohnen-, Soja- und Linsenstärken und Getreidestärken, wie Weizen-, Roggen-, Gersten- sowie Hafer- und Maisstärke.

Als **Monosaccharide** seinen D-Glucose, D-Galactose, D-Mannose, D-Fructose, L-Arabinose, D-Xylose, D-Ribose und 2-Desoxy-D-ribose genannt, besonders bevorzugt unter den Monosacchariden sind D-Glucose und D-Fructose.

Als **Disaccharide** seien genannt Cellobiose, Maltose (Malzzucker), Lactose (Milchzucker) und Saccharose (Rohrzucker). Weitere Disaccharide sind Gentobiose, Melibiose, Trehalose und Turanose. **Pentosane** sind aus Pentosen aufgebaute Polyosen.

Der Gehalt an partikelförmigem Träger beträgt in der Regel 40 bis 90 Gew.- % -bezogen auf das Gesamtgewicht der Mischung, insbesondere 60 bis 80 Gew.-%.

In einer weiteren Ausführungsform der Erfindung werden als partikelförmige Träger Proteine eingesetzt. Als pflanzliche Proteine seien genannt: Getreideproteine wie Weizen-, wie Weizen-, Roggen-, Gersten- sowie Hafer- und Maisproteine; Leguminosenproteine wie Erbsen-, Bohnen-, Soja- und Linsenproteine, Knollenproteine, wie Kartoffelproteine. Als tierische Proteine seinen genannt Milchproteine, wie beispielsweise Caseine und Molkenproteine, und Kollagen. Bevorzugt im Sinne der vorliegenden Erfindung ist die Verwendung von pflanzlichen Proteinen.

Die vorliegende Erfindung umfaßt die Erkenntnis, dass alle genannten partikelförmigen Träger in Mischungen untereinander eingesetzt werden können.

Die partikelförmigen Trägersubstanzen pflanzlichen Ursprungs können als weitere Bestandteile Pflanzenfasern enthalten. In der Regel liegt der Anteil dieser Pflanzenfasern bei 1 bis 10 Gew.-% bezogen auf die partikelförmigen Träger.

Als weitere Bestandteile können die partikelförmigen Träger beispielsweise Fette, Antioxidantien sowie Mineralsalze enthalten. In der Regel liegt der Anteile an diesen weiteren Stoffe bei 1 bis 10 Gew.-% bezogen auf die partikelförmigen Träger.

### Verfahren

Üblicherweise wird der partikelförmige Träger in einer Mischvorrichtung vorgelegt. Als Mischvorrichtung sind alle dem Fachmann bekannten Mischer geeignet, wie beispielsweise Pulvermischer vom Typ Lödige. Als besonders vorteilhaft hat es sich erwiesen Schneckenmischer einzusetzen. Zum vorgelegten Träger erfolgt die Zugabe der oberflächenaktiven Substanz. Diese Zugabe kann entweder oberhalb oder unterhalb des Schmelzpunktes der oberflächenaktiven Substanz erfolgen. Gibt man die oberflächenaktive Substanz unterhalb ihres Schmelzpunktes zu, ist es sinnvoll die Mischung über den Schmelzpunkt der oberflächenaktiven Substanz zu erwärmen, um eine ausreichende Mischung mit dem Träger zu erzielen. Die oberflächenaktive Substanz kann auch in flüssigem Zustand zugegeben werden. Die Zugabe der oberflächenaktiven Substanz zum Träger kann sowohl einstufig als auch mehrstufig erfolgen. Bevorzugt im Sinne der Erfindung ist die mehrstufige Zugabe. In der Regel wird beim Mischen eine Temperatur von unter 100 °C eingehalten, dies ist insbesondere bei temperaturempfindlichen oberflächenaktiven Substanzen von Vorteil.

Der Wassergehalt der so hergestellten Mischung kann nun durch Zugabe von Wasser auf einen Gesamtwassergehalt von 1 bis 15 Gew.-%, insbesondere von 1 bis 10 Gew.-% - bezogen auf das Pulverprodukt - eingestellt werden.

Anschließend wird das Mischgut vermahlen. Es hat sich als vorteilhaft erwiesen, das Mischgut kalt zu vermahlen und eine Temperatur von 50 °C, insbesondere von 30 °C, nicht zu überschreiten. Zum Kaltvermahlen sind prinzipiell alle Mühlentypen geeignet, die es ermöglichen die Mahltemperatur zu kontrollieren. Das Kaltvermahlen des Mischgutes kann beispielsweise wie folgt durchgeführt werden: das Mischgut wird über ein Dosiergerät in einen Wirbelschneckenkühler mit Stickstoffeinspeisung geführt und von dort in eine Prall- bzw. Feinprallmühle eingespeist. Durch diese Mühlen lassen sich Temperaturen von unter 10 °C am Mühlenaustritt erreichen. Neben dieser Kaltvermahlung mit Stickstoff können auch anderen Mahlanlagen, wie sie z.B. beim Mahlen von sehr wärmeempfindlichen Wachsen eingesetzt werden verwendet werden, beispielsweise seien hier genannt: Fließbett- oder Gegenstrahlmühlen, wie sie z.B. unter der Bezeichnung Alpine AFG-R im Handel erhältlich sind. Bei dieses Mühlentypen ist eine Vermahlung bei 20 bis 21 °C möglich. In einer bevorzugten Ausführungsform der vorliegenden Erfindung wird die Mischung in einer Prallmühle, einer Feinprallmühle oder einer Sichtermühle vermahlen. Als besonders geeignet hat sich hierbei eine Mahltrocknungsanlage vom Typ ACM von Hosokawa MikroPull erwiesen.

Ein weiterer Gegenstand der Erfindung betrifft die Verwendung der nach dem erfindungsgemäßen Verfahren hergestellten Produkte zur Herstellung von Aufschlagmittel.

Ein weiterer Gegenstand der Erfindung betrifft die Verwendung der nach dem erfindungsgemäßen Verfahren hergestellten Produkte zur Herstellung von Backmitteln.

### Beispiele

### Beispiel 1

### a) Mischen

In einem kontinuierlichen, heizbaren Schneckenmischer wurde 19,5 kg Reisstärke vorgelegt. Hierzu wurden 10,5 kg einer Mischung Polyglycerinester (Polyglycerinester von Speisefettsäuren EC Nr. E 475 Mono [Polymuls 2G® Henkel KGaA]) und Glyceriden (Mono- und Diglyceriden aus Speisefettsäuren EC Nr. E 471 [Monomuls 90-55 ® Henkel KGaA]) zugegeben. Diese Mischung enthielt 65 Gew.-% Polyglycerinester und 35 Gew.-% Glyceride. Der Gehalt an oberflächenaktiver Substanz betrug 35Gew.-% bezogen auf die Gesamtmischung. Die Produkttemperatur der Mischung im Schneckenmischer wurde von 80 °C auf 100 °C am Ausgang des Schneckenmischers erhöht.

### b) Vermahlen

Das grobkörnige Produkte der Mischung wurde in einer Mahltrocknungsanlage vom Typ ACM 30 (Hosokawa MikroPull) kalt vermahlen (Temperatur beim Mühlenaustritt betrug 25 °C). Auf diese Weise wurde ein feines, frei fließendes Produkt erhalten. Über 75% der Partikel weisen einen Durchmesser unter 250 µm auf.

### Vergleichsbeispiel

Als Vergleichsbeispiel wurde ein Aufschlagmittel durch Extrusion, wie in EP 153 870 (Nexus) beschrieben, hergestellt. Dazu wurden in einem Extruder (ZKS 40, Fa. Werner & Pfleiderer) 19,5 kg Reisstärke vorgelegt. Hierzu wurden 10,5 kg einer Mischung Polyglycerinester (Polyglycerinester von Speisefettsäuren EC Nr. E 475 Mono [Polymuls 2G® Henkel KGaA]) und Glyceriden (Mono- und Diglyceriden aus Speisefettsäuren EC Nr. E 471 [Monomuls 90-55 ® Henkel KGaA]) zugegeben. Diese Mischung enthielt 65 Gew.-% Polyglycerinester und 35 Gew.-% Glyceride. Der Gehalt an oberflächenaktiver Substanz betrug 35 Gew.-% bezogen auf die Gesamtmischung. Die Produkttemperatur der Mischung im Extruder wurde von 80 °C auf 130 °C (am Ende) erhöht. Der Austrag aus dem Extruder erfolgte über eine Düsenplatte (4x2 mm). Die Produkttemperatur betrug, vor der Düse gemessen, 120 °C und der Druck 30 bar. Das extrudierte Produkt wurde direkt als Aufschlagmittel in der anwendungstechnischen Prüfung eingesetzt.

### Anwendungstechnische Prüfung

Zur Untersuchung der Wirksamkeit des Aufschlagmittels wurde folgende Standardrezeptur hergestellt. Das Aufschlagmittel gemäß Beispiel 1 wurde dieser Standardrezeptur zugesetzt. Als Vergleich diente die entsprechende Standardrezeptur mit einem durch Extrusion hergestellten Aufschlag mittel.

### Standardrezeptur

60 g Weizenmehl
40 g Weizenstärke
100 g Ei
82 g Zucker
40 g Wasser
4 g Backpulver
11 g Aufschlagmittel

Das Aufschlagmittel wurde mit den anderen Zutaten unmittelbar vor dem Aufschlagen gemischt und dann für 5 Minuten in einem Mixer aufgeschlagen. Anschließend wurde das Massengewicht einer definierten Volumenmasse bestimmt. Es betrug für das erfindungsgemäße Beispiel 336 g/l, für das Vergleichsbeispiel 340 g/l.

## Patentansprüche

1. Verfahren zur Herstellung eines frei fließfähigen Pulverproduktes, bei dem man eine oberflächenaktive Substanz mit einem partikelförmigen Träger mischt und anschließend vermahlt.

2. Verfahren nach Anspruch 1, **dadurch gekennzeichnet,** daß die oberflächenaktive Substanz ausgewählt ist aus der Gruppe, die gebildet wird von Nahrungsmittelemulgatoren und Emulgatoren für kosmetische und/oder pharmazeutische Mittel.

3. Verfahren nach den Ansprüchen 1 und/oder 2, **dadurch gekennzeichnet,** daß der partikelförmigen Träger einen Träger pflanzlichen Ursprungs ist.

4. Verfahren nach mindestens einem der vorangegangenen Ansprüche, **dadurch gekennzeichnet,** daß man als partikelförmige Träger Stoffe einsetzt, die ausgewählt sind aus der Gruppe, die gebildet wird von pflanzlichen Mehlen, pflanzlichen Stärken, Monosacchariden, Disacchariden und Pentosane.

5. Verfahren nach mindestens einem der vorangegangenen Ansprüche, **dadurch gekennzeichnet,** daß als partikelförmige Träger Proteine eingesetzt werden.

6. Verfahren nach mindestens einem der vorangegangenen Ansprüche, **dadurch gekennzeichnet,** daß der Gehalt der oberflächenaktiven Substanz 10 bis 60 Gew.-%-bezogen auf das Gesamtgewicht der Mischung- beträgt.

7. Verfahren nach mindestens einem der vorangegangenen Ansprüche, **dadurch gekennzeichnet,** daß der Gehalt der partikelförmigem Träger 40 bis 90 Gew.-% -bezogen auf das Gesamtgewicht der Mischung- beträgt.

8. Verfahren nach mindestens einem der vorangegangenen Ansprüche, **dadurch gekennzeichnet,** daß die Temperatur beim Mischen unterhalb von 100 °C liegt.

9. Verfahren nach mindestens einem der vorangegangenen Ansprüche, **dadurch gekennzeichnet,** daß die Temperatur beim Vermahlen unterhalb von 50 °C liegt.

10. Verfahren nach mindestens einem der vorangegangenen Ansprüche, **dadurch gekennzeichnet,** daß die Mischung in einer Prallmühle vermahlen wird.

11. Verwendung des nach den Ansprüchen 1 bis 10 hergestellten freifließfähigen Pulverproduktes zur Herstellung von Aufschlagmitteln.

12. Verwendung des nach den Ansprüchen 1 bis 10 hergestellten freifließfähigen Pulverproduktes zur Herstellung von Backmitteln.
